**Europäisches Patentamt**

**European Patent Office**

**Office européen des brevets**

(11) Veröffentlichungsnummer: **0 017 951**
**A1**

# EUROPÄISCHE PATENTANMELDUNG

(21) Anmeldenummer. **80101992.8**

(22) Anmeldetag **14.04.80**

(51) Int. Cl.³: **A 61 K 7/46, C 11 D 1/08**

(30) Priorität. **20.04.79 DE 2915948**

(43) Veröffentlichungstag der Anmeldung: **29.10.80**
**Patentblatt 80/22**

(84) Benannte Vertragsstaaten **AT BE CH DE FR GB IT LI NL SE**

(71) Anmelder: **Henkel Kommanditgesellschaft auf Aktien, -Patentabteilung- Postfach 1100 Henkelstrasse 67, D-4000 Düsseldorf 1 (DE)**

(72) Erfinder: **Salz, Rainer, Dr., Itterstrasse 33, D-4000 Düsseldorf 13 (DE)**
Erfinder. **Scheuermann, geb. Esano-Solomon, Fanny, Dr., Volmerswerther Strasse 66, D-4000 Düsseldorf (DE)**
Erfinder: **Glasl, Johann, Dr., Baverter Strasse 58, D-5650 Solingen (DE)**

(54) **Verwendung von Alkylenbernsteinsäurederivaten zur Solubilisierung von Parfümölen in Lösungen mit hohem Elektrolytgehalt.**

(57) Verwendung von Alkylenbernsteinsäuresalzen beziehungsweise Alkylenbernsteinsäuremonoestern mit ein- oder mehrwertigen Alkoholen mit 1–4 Kohlenstoffatomen als Esterkomponente sowie deren Salzen, deren Alkylenkette 8 bis 16 Kohlenstoffatome enthält, als hydrotrope Substanzen in Kombination mit üblichen Lösungsvermittlern, zur Solubilisierung von Parfümölen in wäßrigen Lösungen mit hohem Elektrolytgehalt.

EP 0 017 951 A1

Henkelstraße 67
4000 Düsseldorf, den 18.4.1979

HENKEL KGaA
ZR-FE/Patente
z-sü

P a t e n t a n m e l d u n g

D 5803

"Verwendung von Alkylenbernsteinsäurederivaten zur Solubilisierung von Parfümölen in Lösungen mit hohem
Elektrolytgehalt"

Die Erfindung betrifft die Verwendung von Alkylenbernsteinsäuresalzen beziehungsweise Alkylenbernsteinsäuremonoestern mit ein- oder mehrwertigen Alkoholen mit
1 - 4 Kohlenstoffatomen, deren Alkylenkette 8 - 16
Kohlenstoffatome enthält, als hydrotrope Substanzen in
Kombination mit üblichen Lösungsvermittlern zur Solubilisierung von Parfümölen in Lösungen mit hohem
Elektrolytgehalt.

Zur Parfümierung klarer, wäßriger Lösungen können in
einigen Fällen wasserlösliche Parfümöle verwendet werden.
Der Großteil der ätherischen Riechstoffe, Parfümöle und
Aromen sind jedoch öllösliche Produkte, die erst durch
den Zusatz sogenannter Lösungsvermittler in klare,
stabile, wäßrige Lösung gebracht werden können. Als
Lösungsvermittler werden dabei anionische, kationische
oder nichtionische Tenside mit hoher Hydrophilie verwendet. Die Art und die Einsatzmenge dieser Lösungsvermittler hängt in erster Linie von der Natur des zu
solubilisierenden Parfümöls, aber auch von den anderen
Inhaltsstoffen der Lösung ab. Bei der Einarbeitung

/2

öllöslicher Parfümöle in elektrolythaltige Lösungen
wachsen die Schwierigkeiten jedoch sehr stark an.
Bereits die Zugabe des Tensids allein ohne Parfümöl
kann sich infolge des Aussalzeffektes, durch den das
Tensid aus der Lösung verdrängt wird, als unmöglich
erweisen. Es bedarf daher der Zugabe einer dritten
hydrotropen Substanz, die selbst gut wasserlöslich
ist und gleichzeitig Elektrolytcharakter besitzt, um
zusammen mit dem Lösungsvermittler die Solubilisierung
des Parfümöles zu erreichen.

Als hydrotrope Substanzen werden zum Beispiel Arylsulfonate wie Cumol-, Benzol-, Toluolsulfonat oder
$(C_1-C_8)$-Alkyl-monoglykolethersulfate eingesetzt. Bei
einer Einsatzmenge von 5 - 15 % und in Kombination mit
üblichen Lösungsvermittlern ermöglichen sie die Solubilisierung von Parfümölen in wäßrigen Lösungen, die
bis zu 15 Gewichtsprozent Elektrolyt enthalten.

Liegt der Elektrolytgehalt der Lösung jedoch über
15 Gewichtsprozent, so werden auch diese hydrotropen
Substanzen und zusammen mit diesen die Lösungsvermittler
und Parfümöle aus der Lösung verdrängt. Es bestand
daher die Aufgabe, hydrotrope Substanzen aufzufinden,
die in Kombination mit üblichen Lösungsvermittlern
die Solubilisierung öllöslicher Parfümöle in wäßrigen
Lösungen mit hohem Elektrolytgehalt ermöglichen.

Diese Aufgabe wurde dadurch gelöst, daß man als hydrotrope Substanzen Alkylenbernsteinsäuresalze beziehungsweise Alkylenbernsteinsäuremonoester mit ein- oder
mehrwertigen Alkoholen mit 1 - 4 Kohlenstoffatomen
als Esterkomponente sowie deren Salze, deren Alkylenkette 8 - 16 Kohlenstoffatome enthält, in Kombination
mit üblichen Lösungsvermittlern, zur Solubilisierung

/3

0017951

von Parfümölen in wäßrigen Lösungen mit hohem Elektrolytgehalt verwendet.

Die als hydrotrope Substanzen zur Solubilisierung von
Parfümölen in Lösungen mit hohem Elektrolytgehalt zu
verwendenden Alkylenbernsteinsäurederivate sind nach
bekannten Verfahren, wie sie zum Beispiel von B. Cornils
und P. Schneller in der "Chemiker-Zeitung (1976) Nr. 5,
Seite 203 ff." beschrieben werden, zugänglich. Die Herstellung kann in der Weise erfolgen, daß man ein entsprechendes Olefin mit end- oder innenständiger Doppelbindung und Maleinsäureanhydrid im Molverhältnis 2 : 1
mischt und falls erforderlich, im Autoklaven während
mehrerer Stunden bei 170 - 240° C, in Gegenwart eines
Radikalinhibitors wie Hydrochinon oder eines Phenols
wie zum Beispiel 2,6-Di-tert.-butyl-4-methyl-phenol
umsetzt. Die erhaltenen Alkylenbernsteinsäureanhydride
lassen sich durch einstündiges Kochen in Wasser zu den
freien Säuren hydrolysieren. Durch Neutralisation mit
der berechneten Menge Base lassen sich die Salze gewinnen,
wobei den Alkalisalzen und hier wiederum den Di-Natriumsalzen, die größte Bedeutung zukommt.

Die Monoester der Alkylenbernsteinsäuren sind auf einfachem Wege durch Alkoholyse der entsprechenden Alkylenbernsteinsäureanhydride erhältlich. Die Anhydride reagieren bereits bei Raumtemperatur langsam und mit überschüssigem Alkohol werden die Halbester schon nach
kurzer Zeit (abhängig von der Art des Alkohols) bei
Rückflußtemperatur gebildet. Bei Polyolen wie Ethylenglykol oder Glycerin muß zur Vermeidung von Polymeren in
jedem Fall ein Alkoholüberschuß eingesetzt werden.
Durch Neutralisation der erhaltenen Monoester mit der
berechneten Menge Base erhält man die Salze der Alkylenbernsteinsäuremonoester, wobei wiederum den Natriumsalzen die größte Bedeutung zukommt. Als alkoholische

/4

Komponente der Monoester kommen ein- und mehrwertige aliphatische Alkohole mit 1 - 4 Kohlenstoffatomen in Frage wie zum Beispiel Methanol, Ethanol, Propanol, i-Propanol, Butanol, Ethylenglykol, Glycerin, wobei Methanol, Ethanol und Ethylenglykol die größte Bedeutung zukommt.

Die für die Umsetzung mit Maleinsäureanhydrid erforderlichen Olefine können $\alpha$-Olefine oder Monoolefine mit innenständiger Doppelbindung und einer Kettenlänge von 8 - 16 Kohlenstoffatomen sein. Der bevorzugte Kettenlängenbereich liegt bei 10 - 14 Kohlenstoffatomen. Entsprechende 1,2-Monoolefine sind beispielsweise durch Polymerisation von Ethylen mit organischen Aluminiumverbindungen als Katalysatoren oder durch thermisches Cracken von Paraffinkohlenwasserstoffen zugänglich. Monoolefine mit innenständiger Doppelbindung können durch katalytische Dehydrierung oder durch Chlorierung/Dehydrierung von linearen Paraffinkohlenwasserstoffen oder durch Isomerisierung von $\alpha$-Olefinen hergestellt werden.

In Kombination mit den erfindungsgemäß als hydrotrope Substanzen zu verwendenden Alkylenbernsteinsäurederivaten können als Lösungsvermittler alle für diesen Zweck gebräuchlichen anionischen, kationischen oder nichtionischen Tenside eingesetzt werden, Fettalkoholsulfate, Fettalkoholethersulfate, Alkylsulfonate, Olefinsulfonate, Monofettsäureester von Polyolen, Fettaminpolyglykolether, Alkylphenolpolyglykolether, polyoxethyliertes Rizinusöl. Besondere Bedeutung kommt dabei den nichtionischen Tensiden zu.

/5

Bei den zu parfümierenden Produkten mit hohem
Elektrolytgehalt handelt es sich in erster Linie
um industrielle Reinigungsmittel, Haushaltsreiniger,
Maschinengeschirrspülmittel, Bleichmittel usw.

Die in derartige Produkte in klarer Lösung einzuarbeitende Parfümölmenge wird im allgemeinen um
0,2 bis 0,3 Gewichtsprozent, bezogen auf das gesamte
Produkt, liegen. Höhere Mengen werden schon aus preislichen Gründen kaum in Frage kommen. Um eine klare,
lager- und temperaturbeständige Lösung des Parfümöls in diesen Produkten zu erzielen, ist ein Zusatz
von 4 - 5 Gewichtsprozent der erfindungsgemäß zu
verwendenden Alkylenbernsteinsäurederivate und
1 - 1,5 Gewichtsprozent der üblichen Lösungsvermittler, bezogen auf das gesamte Produkt, erforderlich.

Sollte aus besonderen Gründen eine Anhebung der
Parfümölmenge bis auf 1 Gewichtsprozent erforderlich
sein, so müßte die Zusatzmenge an Alkylenbernsteinsäurederivat und Lösungsvermittler auf circa 15 Gewichtsprozent beziehungsweise circa 4 Gewichtsprozent erhöht
werden.

Zur Herstellung der parfümierten Lösungen werden
0,2 bis 0,3 Gewichtsteile Parfümöl mit 1 bis 1,2
Gewichtsteilen Lösungsvermittler und 4 - 5 Gewichtsteilen Alkylenbernsteinsäurederivat vermischt und zu
dieser Mischung wird die zu parfümierende elektrolythaltige Lösung in eine zu 100 Gewichtsteilen ergänzenden Menge hinzugegeben. Es werden klare, lager-
und temperaturbeständige Produkte erhalten.

/6

Die nachfolgenden Beispiele sollen den Gegenstand
der Erfindung näher erläutern, ohne ihn jedoch
hierauf zu beschränken.

/7

## B e i s p i e l e

Zur Herstellung der erfindungsgemäß zu verwendenden Alkylenbernsteinsäurederivate wurden die entsprechenden Olefine und Maleinsäureanhydrid im Molverhältnis 2 : 1 gemischt und mit 1 g Hydrochinon pro Mol Olefin versetzt. Die Umsetzung wurde bei 190 - 220$^\circ$ C vorgenommen und bei den kürzerkettigen Olefinen wie $\alpha$-$C_{10}$-Olefin im Autoklaven durchgeführt. Die Daten der erhaltenen Alkylenbernsteinsäureanhydride sind der nachfolgenden Tabelle 1 zu entnehmen.

### Tabelle 1

Analytische Daten der Alkylenbernsteinsäureanhydride

| Ausgangs-olefin | Reaktions-zeit (h) | Ausbeute % | Jod-zahl | Verseifungs-zahl | Fp./Sdp.($^\circ$C) |
|---|---|---|---|---|---|
| $\alpha$-$C_{10}$ | 12 | 64,6 | 106,6 | 471,3 | 137$^\circ$C/0,1 Torr |
| $\alpha$-$C_{12}$ | 15 | 51,5 | 94,0 | 414,9 | 44-45$^\circ$C |
| $\alpha$-$C_{14}$ | 12 | 54,5 | 85,2 | 370,5 | 51-53$^\circ$C |
| $i$-$C_{11/14}$ | 17 | 77,5 | 95,0 | 386,1 | 128-146$^\circ$C/ 0,05 Torr |

Die Hydrolyse der Alkylenbernsteinsäureanhydride zu den entsprechenden freien Säuren wurde durch einstündiges Kochen mit Wasser bewirkt. Die analytischen Daten der Säuren sind in der folgenden Tabelle 2 angeführt.

/8

0017951

Tabelle 2

Analytische Daten der Alkylenbernsteinsäuren

| Ausgangs-olefin | Jodzahl | Säurezahl | Fp. $^{o}$ C |
|---|---|---|---|
| $\alpha\text{-}C_{10}$ | 96,5 | 440,0 | Wachs |
| $\alpha\text{-} C_{12}$ | 83,4 | 393,6 | 73 - 74 |
| $\alpha\text{-}C_{14}$ | 80,3 | 359,9 | 75 - 76 |
| $i\text{-}C_{11/14}$ | 85,5 | 367,5 | zähe Flüssig-keit |

Die Di-Natriumsalze der Alkylenbernsteinsäuren wurden durch Umsetzung einer berechneten Menge Natronlauge mit den entsprechenden Alkylenbernsteinsäureanhydriden hergestellt. Dazu wurde die Lauge vorgelegt und das Anhydrid zudosiert. Danach wurde 30 Minuten lang bei $60 - 70^{o}$ C gerührt und anschließend das Wasser im Vakuum abgedampft. In der Tabelle 3 sind die Löslichkeiten der Salze angeführt.

Tabelle 3

Löslichkeiten der Di-Natriumsalze der Alkylenbernstein-säuren in $H_2O$ bei Raumtemperatur

| Ausgangsolefin | $\alpha\text{-}C_{10}$ | $\alpha\text{-}C_{12}$ | $\alpha\text{-}C_{14}$ | $i\text{-}C_{11/14}$ |
|---|---|---|---|---|
| Löslichkeit in Gewichts-prozent | 10 | 5 | 0,5 | 25 |

Zur Herstellung der Alkylenbernsteinsäuremonoester wurden die Reaktionskomponenten Alkylenbernsteinsäureanhydrid und Alkohol im Molverhältnis 1 : 1,3 gemischt und erhitzt. Es wurde

/9

2 Stunden lang weitergerührt und hernach der überschüssige Alkohol abdestilliert. Die Monoester fielen in Form leicht viskoser, gelber Flüssigkeiten an, die sich in organischen Lösungsmitteln, jedoch nicht in Wasser lösen. Eine Destillation der Monoester war nicht möglich, da sie im Vakuum bei Temperaturen über $150^{o}$ C wieder in Alkohol und Anhydrid gespalten werden. Die analytischen Daten der Monoester sind der Tabelle 4 zu entnehmen.

<u>Tabelle 4</u>

Analytische Daten der Alkylenbernsteinsäuremonoester

| Ausgangs-olefin | Alkohol | Jodzahl | Säurezahl | Molgewicht aus Jodzahl |
|---|---|---|---|---|
| $\alpha$-C$_{10}$ | Methanol | 92,5 | 179,8 | 274,4 |
| $\alpha$-C$_{10}$ | n-Propanol | 83,3 | 160,9 | 304,6 |
| $\alpha$-C$_{12}$ | Methanol | 83,9 | 170,7 | 302,5 |
| $\alpha$-C$_{12}$ | n-Propanol | 76,1 | 148,9 | 333,5 |
| $\alpha$-C$_{14}$ | Methanol | 78,5 | 152,2 | 323,3 |
| $\alpha$-C$_{14}$ | n-Propanol | 71,4 | 126,3 | 355,5 |
| i-C$_{11/14}$ | Methanol | 86,4 | 179,2 | 293,8 |
| i-C$_{11/14}$ | n-Propanol | 77,1 | 164,5 | 329,2 |

Die Herstellung der Natriumsalze der Alkylenbernsteinsäuremonoester erfolgte durch Einrühren der Monoester in eine berechnete Menge 5 %iger Natronlauge.

Für den Einsatz in den nachstehend beschriebenen
Beispielen wurden folgende Produkte ausgewählt:

A) $\alpha$-$C_{10}$-Alkylenbernsteinsäure-di-Natriumsalz.

B) $\alpha$-$C_{12}$-Alkylenbernsteinsäure-di-Natriumsalz.

C) i-$C_{11-14}$-Alkylenbernsteinsäure-di-Natriumsalz.

D) $\alpha$-$C_{10}$-Alkylenbernsteinsäure-mono-Natriumsalz-
   monomethylester.

E) $\alpha$-$C_{12}$-Alkylenbernsteinsäure-mono-Natriumsalz-
   monomethylester.

F) i-$C_{11-14}$-Alkylenbernsteinsäure-mono-Natriumsalz-
   monomethylester.

G) $\alpha$-$C_{12}$-Alkylenbernsteinsäuremonomethylester.

Als zu solubilisierende Parfümöle dienten für die
Testversuche folgende Substanzen:

01 Rosmarinöl          06 Orangenöl

02 Bergamottöl         07 Pineöl

03 Cedernholzöl        08 Pfefferminzöl

04 Nelkenblätteröl     09 Patschouliöl

05 Zitronenöl          010 Lavandinöl

Zur Durchführung der Versuche wurden jeweils

        0,2 g der Parfümöle 01 - 010 mit

        1,0 g Fettaminpolyglykolether (Araphen T 100$^{(R)}$)
                als Lösungsvermittler und

        4,0 g der obengenannten Alkylenbernsteinsäure-
                derivate als hydrotrope Substanzen verrührt.

Zu jeder einzelnen der auf diese Weise erhaltenen
70 Proben wurden unter Rühren 94,8 g einer Natriumhypochloritlösung gegeben, die durch Lösen von 5 kg

/11

Natriumhypochlorit in 4,48 Litern Wasser erhalten worden war. Es wurden in allen Fällen klare Lösungen der Parfümöle erhalten, die selbst nach 48-stündiger Lagerung bei + 40° C und -6° C klar blieben und keine Öltröpfchen an der Oberfläche abschieden.

In einem weiteren Beispiel wurden

    0,3 g Zitronenöl
    1,2 g Nonylphenolpolyglykolether (Eumulgin 286[R])
    5,0 g $\alpha$-$C_{10}$-Alkylenbernsteinsäure-di-Natriumsalz

zusammengerührt und anschließend mit einer Lösung aus

    55,0 g Natriumhypochlorit in
    38,5 g Wasser

innig vermischt. Auch in diesem Fall wurde eine klare, stabile Lösung des Parfümöls erhalten. Ohne daß das Ergebnis beeinträchtigt wird, läßt sich das Zitronenöl durch ein anderes Parfümöl und das $\alpha$-$C_{10}$-Alkylenbernsteinsäure-di-Natriumsalz durch eine andere der vorstehend genannten erfindungsgemäßen hydrotropen Substanzen ersetzen.

In Vergleichsversuchen wurden in der Rezeptur

    0,3 g Zitronenöl
    1,2 g Nonylphenolpolyglykolether (Eumulgin 286[R])
    5,0 g hydrotrope Substanz
    55,0 g Natriumhypochlorit
    38,5 g Wasser

anstelle des $\alpha$-$C_{10}$-Alkylenbernsteinsäure-di-
Natriumsalzes als hydrotrope Substanzen folgende
Produkte eingesetzt:

Octen-1-sulfonat, Natriumsalz, Cumolsulfonat, Natriumsalz, Benzolsulfonat, Natriumsalz, Ethylhexanolsulfat, Natriumsalz

Dimethylkokosaminoxid,

Na-$\alpha$-sulfo-$C_8$-säure-methylester,

$C_{14/16}$-$\alpha$-olefinsulfonat, Natriumsalz,

Sulfobernsteinsäuremethylester, Natriumsalz, Diisooctylsulfosuccinat-Natriumsalz.

In keinem Fall ließ sich eine klare, stabile Lösung des
Parfümöls in der hochelektrolythaltigen Lösung erzielen.

Zur Bereitung von Bleich-, Reinigungs- und Spülmitteln
mit hohem Elektrolytgehalt, die eine Parfümierung
durch Parfümöle erhalten sollen, wird zunächst eine
Mischung aus

0,2 - 0,3 Gewichtsteilen Parfümöl

1,0 - 1,2       "     Lösungsvermittler anionischer oder nichtionischer
Art

4,0 - 5,0       "     Alkylenbernsteinsäurederi-
vat

hergestellt und diese Mischung hernach unter Rühren
mit dem zu parfümierenden elektrolythaltigen Mittel
unter Ergänzung zu 100 Gewichtsteilen versetzt.

"Verwendung von Alkylenbernsteinsäurederivaten zur Solubilisierung von Parfümölen in Lösungen mit hohem Elektrolytgehalt"

Patentansprüche:

1. Verwendung von Alkylenbernsteinsäuresalzen beziehungsweise Alkylenbernsteinsäuremonoestern mit ein- oder mehrwertigen Alkoholen mit 1 - 4 Kohlenstoffatomen als Esterkomponente sowie deren Salzen, deren Alkylenkette 8 - 16 Kohlenstoffatome enthält, als hydrotrope Substanzen in Kombination mit üblichen Lösungsvermittlern, zur Solubilisierung von Parfümölen in wäßrigen Lösungen mit hohem Elektrolytgehalt.

2. Verwendung von Alkylenbernsteinsäuresalzen und Alkylenbernsteinsäuremonoestersalzen nach Anspruch 1, dadurch gekennzeichnet, daß die Natriumsalze eingesetzt werden.

3. Verwendung von Alkylenbernsteinsäuresalzen, Alkylenbernsteinsäuremonoestern und deren Salzen nach Anspruch 1 und 2, dadurch gekennzeichnet, daß diese in den zu parfümierenden wäßrigen Lösungen mit hohem Elektrolytgehalt in einer Menge von 4 - 5 Gewichtsprozent, neben 1 - 1,5 Gewichtsprozent an üblichen Lösungsvermittlern und 0,2 - 0,3 Gewichtsprozent an zu solubilisierendem Parfümöl, alles bezogen auf das gesamte Endprodukt, eingesetzt werden.

| | Europäisches Patentamt | **EUROPÄISCHER RECHERCHENBERICHT** | 0017951 |
|---|---|---|---|

EP 80 10 1942

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE A - 1 956 671 (ROHM & HAAS) <br><br> * Die Patentansprüche; Seite 1, Absatz 2 bis Seite 5, Absatz 2 * <br><br> --- | 1 |
| A | DE - A - 2 255 571 (UNILEVER) <br><br> * Die Patentansprüche; Seite 8, Absatz 2 * <br><br> --- | 1 |
| A | DE - A - 1 939 973 (UNILEVER) <br><br> * Die Patentansprüche * <br><br> --- | 1 |
| A | FR - A - 2 296 683 (LANKRO CHEM.) <br><br> * Die Patentansprüche; Seite 2, Zeile 33 bis Seite 4, Zeile 5 * <br><br> --- | 1 |
| A | US - A - 2 380 699 (L.P. KYRIDES) <br><br> * Die Patentansprüche; Seite 1, Spalte 2, Zeile 37 bis Seite 2, Spalte 2, Zeile 50; Seite 5, Spalte 1, Zeile 37 bis Seite 5, Spalte 2, Zeile 13 * <br><br> --- | 1 |
| A | US - A - 2 360 426 (L.P. KYRIDES) <br><br> * Die Patentanpsrüche * <br><br> --- <br><br> ./. | 1 |

**KLASSIFIKATION DER ANMELDUNG (Int C**

A 01 K 7/40
C 11 D 1/08

**RECHERCHIERTE SACHGEBIETE (Int Cl**

A 01 K 7 40
C 11 D 1/08

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angeführtes Dokument
L: aus andern Grunden angeführtes Dokument
&: Mitglied der gleichen Patentfamilie, übereinstimmendes Dokument

| | Der vorliegende Recherchenbericht wurde für alle Patentansprüche erstellt |
|---|---|

| Recherchenort | Abschlußdatum der Recherche | Prüfer |
|---|---|---|
| DEN HAAG | 27.06.1980 | VANHECKE |

EPA form 1503.1  06.78

| | EINSCHLÄGIGE DOKUMENTE | | | KLASSIFIKATION DER ANMELDUNG (Int Cl) |
|---|---|---|---|---|
| Kategorie | Kennzeichnung des Dokuments mit Angabe, soweit erforderlich, der maßgeblichen Teile | betrifft Anspruch | | |
| A | <u>DE - C - 754 482</u> (R. ENDRES) | 1 | | |
| | * Die Patentansprüche; Seite 1, Zeilen 1-28 * | | | |
| | ------ | | | |
| | | | | **RECHERCHIERTE SACHGEBIETE (Int. C** |